# EUROPEAN PATENT SPECIFICATION

(11) **EP 0 516 948 B1**
(45) Date of publication and mention of the grant of the patent: **31.05.2000**
(21) Application number: 92106544.7
(22) Date of filing: 15.04.1992
(51) Int. Cl.: G01N 33/68, C12Q 1/28, C12Q 1/34, C12Q 1/68, G01N 33/53, C12Q 1/42, C12Q 1/44, G01N 33/58, G01N 33/535, G01N 21/76

(54) **Chemiluminescent method and compositions**
Chemische Lumineszenzverfahren und Zusammensetzung dafür
Méthode et composition pour luminescence chimique

(30) Priority: 24.05.1991 US 705322
(43) Date of publication of application: 09.12.1992
(73) Proprietor: LUMIGEN, INC., Southfield, Michigan 48034 (US)
(72) Inventor: Akhavan-Tafti, M. Hashem, Sterling Heights, Michigan 48310 (US)
(74) Representative: Jones, Helen Marjorie Meredith

(56) References cited:
- EP-A- 0 087 959
- EP-A- 0 116 454
- EP-A- 0 296 752
- EP-A- 0 361 470
- EP-A- 0 384 271
- WO-A-91/05872
- US-A- 4 931 385
- ANALYTICAL BIOCHEMISTRY vol. 151, 1985, NEW YORK US pages 205 - 209; J.A.MATTHEWS ET AL.: 'Enhanced chemiluminescent method for the detection of DNA dot hybridization assays'

## Description

### BACKGROUND OF THE INVENTION

### 1. Field of the Invention

This invention relates to a method of generating light chemically (chemiluminescence) by the action of an enzyme which generates an enhancer for a second enzyme from a protected enhancer where the second enzyme, in turn, catalyzes a chemiluminescent reaction. The invention also relates to the use of this method to detect the first enzyme. Further, the invention relates to the use of the method to detect and quantitate various biological molecules bound to the first enzyme. For example, the method may be used to detect the first enzyme singly or as a label for haptens, antigens and antibodies by the technique of immunoassay, proteins by Western blotting, DNA and RNA by Southern and Northern blotting, respectively. The method may also be used to detect DNA in DNA sequencing applications.

### 2. Description of the Prior Art

a. Chemiluminescent oxidation of luminol and related compounds. The amino-substituted cyclic acylhydrazides such as luminol and isoluminol react with H₂O₂ and a peroxidase enzyme catalyst (such as horseradish peroxidase, HRP) under basic conditions with emission of light. This reaction has been used as the basis for analytical methods for the detection of H₂O₂ and for metal ions. Luminol and isoluminol may be directly conjugated to a species to be detected. The first chemiluminescent immunoassay using luminol as a label was reported by Schroeder for an assay of biotin (H. R. Schroeder, P. O. Vogelhut, R. J. Carrico, R. C. Boguslaski, R. T. Buckler, Anal. Chem., 48, 1933 (1976)). Several applications of the use of luminol derivatives as labels have been reported since then (H. R. Schroeder in Luminescent Immunoassays: Perspectives in Endocrinology and Clinical Chemistry, M. Serio and M. Pazzagli, Eds., Raven Press, New York, pp 129-146 (1982); M. Pazzagli, G. Messeri, A. L. Caldini, G. Monetti, G. Martinazzo, M. Serio, J. Steroid Biochem., 19, 407 (1983); Bioluminescence and Chemiluminescence New Perspectives, J. Scholmerich, et al, Eds., J. Wiley & Sons, Chichester (1987)). Various enhancers have also been employed in conjunction with the use of luminol to increase the intensity of light emitted. These include D-luciferin (T. P. Whitehead, G. H. Thorpe, T. J. Carter, C. Groucutt, L. J. Kricka, Nature, 305, 158 (1983)) and p-iodophenol and p-phenylphenol (G. H. Thorpe, L. J. Kricka, S. B. Mosely, T. P. Whitehead Clin. Chem., 31, 1335 (1985)).
b. Enzyme-Catalyzed Chemiluminescent Reactions
   1. Enzymatic Triggering of Stabilized 1,2-Dioxetanes. Recently developed thermally stable dioxetanes can be triggered by chemical and enzymatic processes to generate chemiluminescence on demand (A. P. Schaap, U.S. Patent No. 4,857,652; A. P. Schaap, R. S. Handley, B. P. Giri, Tetrahedron Lett., 935 (1987); A. P. Schaap, T. S. Chen, R. S. Handley, R. DeSilva, B. P. Giri, Tetrahedron Lett., 1155 (1987); and A. P. Schaap, M. D. Sandison, R. S. Handley, Tetrahedron Lett., 1159 (1987)). These dioxetanes exhibit several key features: (1) the stabilizing influence of spiro-fused adamantyl groups has been utilized to provide dioxetanes that have "shelf lives" of years at ambient temperature and (2) an electron-rich aromatic group which allows the efficient production of excited states and which produces fluorescence when excited and (3) a protecting group which prevents the chemiluminescent decomposition until removed by an enzyme or other chemical reaction. 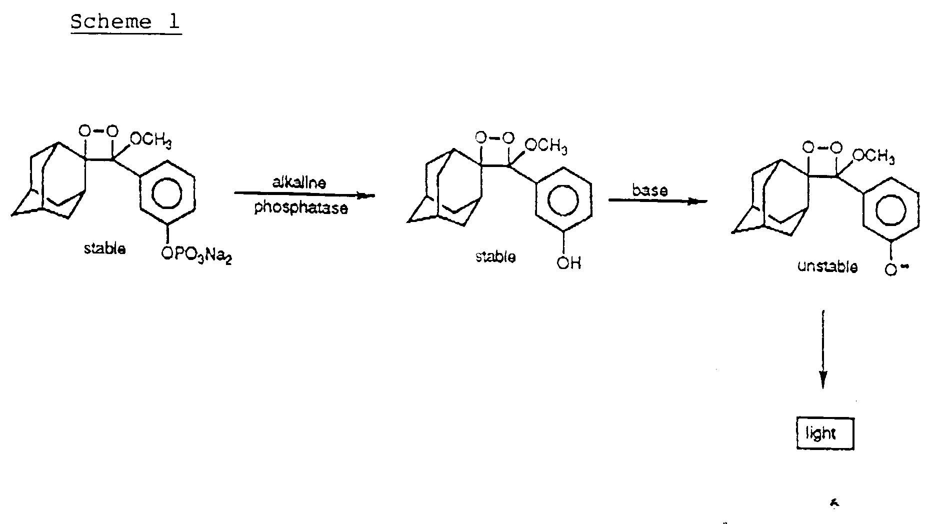
      For example, enzymatic triggering by alkaline phosphatase was observed with the phosphate-substituted dioxetane (1) derived from methyl 3-hydroxybenzoate and adamantanone (Lumigen® PPD). Addition of the enzyme to a solution of dioxetane results in chemiluminescence emitted over several minutes. The total light emission was found to be linearly dependent on the dioxetane concentration. The rate of decay of the emission is a function of enzyme concentration while the total light emission is independent of the enzyme concentration. As a result of the very low background luminescence from slow hydrolysis of the dioxetane in the buffer, in the presence of enhancers less than 10⁻¹⁸ mol (1 attomol) of alkaline phosphatase can be detected (A. P. Schaap, H. Akhavan, L. J. Romano, Clin. Chem., 35, 1863 (1989)). Similar protected dioxetane substrates for other enzymes, namely beta-galactosidase, esterase and sulfatase have also been developed. A number of commercial products for detection of proteins and nucleic acids are sold employing chemiluminescent detection of alkaline phosphatase with Lumigen® PPD (D. Pollard-Knight, A. C. Simmonds, A. P. Schaap, H. Akhavan, M. A. W. Brady, Anal. Biochem., 185, 353-358 (1990); J. M. Clyne, J. A. Running, R. Sanchez-Pescador, D. Besemer, M. Stempien, A. P. Schaap, R. S. Stephens, M. S. Urdea, J. Biolumin. Chemilumin. 2, 193 (1988)).
   2. Enzymatic generation of hydrogen peroxide. Various enzymatic reaction schemes are known which produce hydrogen peroxide. The generated hydrogen peroxide can, in turn, be used to oxidize a compound which emits light. For example, glucose oxidase reacts with O₂ and glucose to produce H₂O₂. Similarly, amino acid oxidase reacts with an amino acid and O₂ to produce H₂O₂. Examples of compounds which are oxidized by hydrogen peroxide to produce light are luminol and isoluminol, lucigenin, esters of N-methylacridine and esters and amides of oxalic acid. Glucose-6-phosphate dehydrogenase and galactose-6-phosphate dehydrogenase have been used to produce H₂O₂ indirectly by reduction of oxygen through an electron-relay system (K. Tanabe, T. Kawasaki, M. Maeda, A. Tsuji, M. Yabuuchi, Bunseki Kagaku, 36, 82 (1987); A. Tsuji, M. Maeda, H. Arakawa, Anal. Sci., 5, 497 (1989)).
   3. Enzymatic generation of luminol from a luminol-NAG conjugate. The compound o-aminophthalydrazide-N-acetyl-beta-D-glucosaminide (luminol-NAG) is a substrate for the enzyme N-acetyl-beta-D-glucosaminidase which serves as a masked form of luminol. Upon action of the enzyme on this substrate, luminol is liberated which may be detected as described above (K. Sasamoto, Y. Ohkura, Chem. Pharm. Bull., 38(5), 1323 (1990)).
   4. Bioluminescent detection of firefly or bacterial luciferase. A class of enzymes known as luciferases, catalyze the autooxidation of certain substrates known as luciferins in a number of living organisms. An example is the firefly whose bioluminescent process oxidizes its luciferin to produce light with 88% efficiency. A DNA dot-hybridization assay has been reported in which alkaline phosphatase was employed to generate free firefly luciferin from the phosphate salt. Luciferase added in the final step caused the chemiluminescent oxidation of the luciferin (R. Huber, R. Geiger, Nuc. Ac. Res., 16(3), 1213 (1988)). Only one enzyme amplification step is involved in this method since the first enzyme only generates a substrate for the second enzyme.
c. Use of Chemiluminescence in Enzyme Immunoassays and DNA Hybridization Assays. Biological assays such as enzyme immunoassays and DNA probe assays involving enzymes utilize a wide variety of substrates which either form a color (chromogenic), become fluorescent (fluorogenic) or emit light (chemiluminogenic) upon reaction with the enzyme. Of these three choices, chemiluminescence offers the greatest sensitivity. In an assay, the enzyme (reporter enzyme) is conjugated or bound to the molecule to be detected or to some other substance capable of selectively binding or associating with the molecule to be detected. Once the bound reporter enzyme is separated from unbound enzyme, a substrate is provided with which the reporter enzyme generates a signal. Chemiluminogenic substrates used to date include enzyme-triggerable dioxetanes such as the alkaline phosphatase substrate Lumigen® PPD. This substrate has been used extensively in enzyme-linked immunoassays and DNA probes. The enzyme horseradish peroxidase has been widely used in enzyme immunoassays and DNA hybridization assays with chemiluminescent detection using luminol or isoluminol as substrate (T. P. Whitehead, G. H. Thorpe, T. J. Carter, C. Groucutt, L. J. Kricka, Nature 305, 158 (1983); G. H. Thorpe, L. J. Kricka, S. B. Mosely, T. P. Whitehead, Clin. Chem., 31 1335 (1985); G. H. Thorpe, S. B. Mosely, L. J. Kricka, R. A. Stott, T. P. Whitehead, Anal. Chim. Acta, 170, 107 (1985); J. A. Matthews, A. Batki, C. Hynds, L. J. Kricka, Anal. Biochem., 151, 205, (1985)). Commercially available kits for conjugation of HRP with enhanced luminol chemiluminescent detection are sold under the tradename Amerlite™.
d. Enzyme cascade sequences for sensitive detection. A coupled enzyme cascade reaction for the colorimetric detection of alkaline phosphatase has been reported (D. M. Obzansky, D. M. Severino, Abstracts of 23rd Oak Ridge Conference on Advanced Analytical Concepts for the Clinical Laboratory, April 12, 1991). In this scheme, alkaline phosphatase generates a substance which reacts with an inactive form of a second enzyme, converting it to its active state. The second enzyme reacts with its own substrate producing H₂O₂. The H₂O₂ is detected through a subsequent colorimetric procedure.
   A colorimetric enzyme immunoassay method has been reported wherein HRP bound in a sandwich immunoassay catalyzes the oxidative deposition of a biotin-phenol conjugate with H₂O₂. The oxidized product becomes deposited on the solid surface of the assay vessel. The bound biotin is used to capture additional enzyme-streptavidin conjugate. The captured enzyme may be either more HRP or a different enzyme, i.e. alkaline phosphatase or beta-galactosidase. No chemiluminescent detection was used (M. N. Bobrow, T. D. Harris, K. J. Shaughnessy, G. J. Litt, J. Immun. Meth., 125, 279 (1989)).
   A method is known which features the enzymatic release of an enzyme inhibitor Anal. Biochem., 179, 229 (1989). The basis of this method is the enzymatic release of an inhibitor to a second enzyme. Sensitivity shows an inverse dependence on concentration of the first enzyme and is limited to the sensitivity with which the second enzyme can be detected. No true amplification is achieved by the use of two enzymes.
   Glucose-6-phosphate dehydrogenase and galactose-6-phosphate dehydrogenase have been used to produce H₂O₂ indirectly by reduction of oxygen through an electron-relay system (K. Tanabe, T. Kawasaki, M. Maeda, A. Tsuji, M. Yabuuchi, Bunseki Kagaku, 36, 82 (1987); A. Tsuji, M. Maeda, H. Arakawa, Anal. Sci., 5, 497 (1989)). In these procedures, enhancement results only from the rapid recycling of the enzyme, not from the generation of another catalytically active species.
   U.S. Patent No. 4,318,980 discloses a heterogeneous binding assay employing a catalytic cycling agent (nicotinamide adenine dinucleotide, NAD) as the label for the analyte. The cycling agent is subsequently detected by a chemiluminescent reaction. Only a single enzyme amplification step results.
   U.S. Patent No. 4,598,042 discloses an immunoassay for phosphatase conjugates wherein the phosphatase converts NADP to NAD. The NAD formed while cycling between its oxidized and reduced forms acts as a cofactor for alcohol dehydrogenase cycling agent and catalyzes the formation of a colored compound. The use of chemiluminescence or peroxidases is not disclosed.
   U.S. Patent 4,498,042 discloses an immunoassay which uses as a label an enzyme which converts an inactive form of a second enzyme to its active form. The second enzyme is detected by reaction with a substrate. Alternatively the second enzyme may react to generate a third enzyme which is subsequently detected by reaction with a substrate.
e. Enhanced Chemiluminescent Assay Procedures.
   WO 91/05872 describes an enhanced chemiluminescent assay, in which a dihydrophthalazinedione such as luminol, a peroxidase such as horseradish peroxidase and an oxidant such as H₂O₂ are co-reacted in the presence of an enhancer such as *p*-iodophenol, is modified. The enhancer is generated by enzyme-catalyzed reaction of a pro-enhancer, e.g. *p*-iodophenol phosphate is cleaved by alkaline phosphatase, enabling this enzyme to be assayed instead of peroxidase. No suppressing agent to retard chemiluminescent generation is disclosed.
f. Enzyme Immunoassays and Immunologic Reagents.
   US 4,931,385 discloses a method of detecting bindable substances such as antibodies and antigens using enzyme linked immunosorbant assays. An antibody coated support is treated with an improved blocking solution containing a blocking agent in admixture with a sugar. The blocking agent prevents non-specific binding of immunologic reagents to a solid support coated with antibody. No chemiluminescent retarding properties are disclosed.

### 3. Summary

The detection of chemiluminescence from the oxidation of an amino-substituted cyclic acylhydrazide by a peroxide compound catalyzed by a peroxidase enzyme can be accomplished with good sensitivity. Enhancement of this reaction by incorporation of an enhancer has permitted the measurement of chemiluminescence using still lower levels of the peroxidase enzyme. Coupling this enzyme to a biological molecule of interest then permits the detection of this biological molecule with great sensitivity. This technology limits the utility specifically to the use of only a peroxidase enzyme. A need exists to make use of this enhanced catalytic generation of amino-substituted cyclic acylhydrazide chemiluminescence by initiation with other enzymes. By making another enzyme the detection marker or label in this system, the result is a further amplification of the enzyme-amplified chemiluminescent signal. This amplification-of-amplification will lead to detection of increasingly lower amounts of enzyme and, hence, to the biological molecule of interest. A key consideration in developing ultrasensitive detection systems is to maintain the lowest possible level of background signal in relation to the signal to be measured. It is of paramount importance in any proposed detection method to provide the largest signal possible through amplification without a concomitant increase in background signal (i.e. only the signal of interest should be amplified).

### OBJECTS

It is therefore an object of the present invention to provide a method and enzyme-triggerable protected enhancers for use in generating chemiluminescence by the action of a peroxidase enzyme for the detection of biological materials and compounds. Additionally, it is an object of the present invention to provide a method and enzyme-triggerable protected enhancers for use in generating chemiluminescence by the action of a peroxidase enzyme for use in immunoassays in solution. Further, it is an object of the present invention to provide a method and enzyme-triggerable protected enhancers for use in generating chemiluminescence by the action of a peroxidase enzyme for detection of proteins in Western blots and DNA in Southern blots and other DNA hybridization assays.

### IN THE DRAWINGS

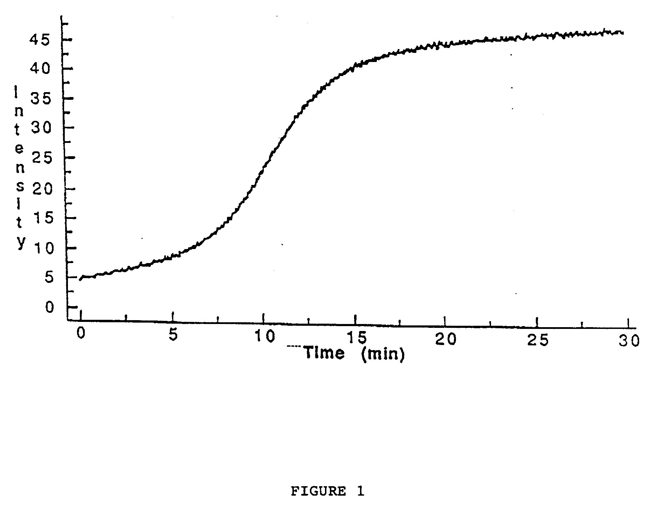

Figure 1 is a plot of chemiluminescence intensity as a function of time using the reaction system described in Example 1. Chemiluminescence was measured in a Turner Designs model TD20-e luminometer at ambient temperature. Light was attenuated with a 200x neutral density filter. The reaction was initiated by addition of alkaline phosphatase.

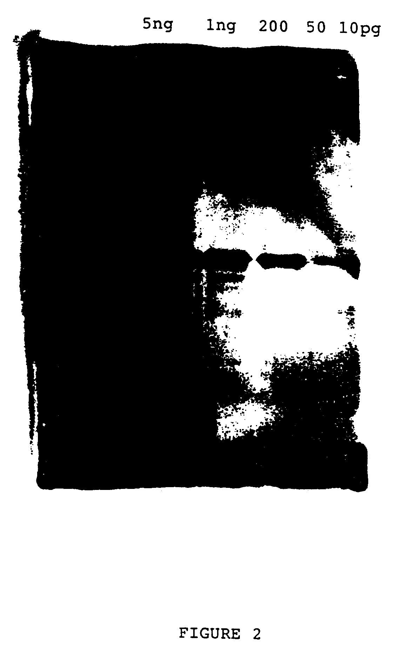

Figure 2 shows chemiluminescent Western blot of human transferrin. Experimental conditions for the Western blot are described in the text. Tracks were loaded with (left to right) 5 ng, 200 pg, 50 pg, and 20 pg human transferrin. Washed and blocked membrane was incubated in the reagent for 5 minutes and exposed to KODAK X-OMAT AR film for 5 seconds.

### GENERAL DESCRIPTION

The present invention relates to a method for generating chemiluminescence which comprises mixing:
(a) a hydrolytic enzyme; and
(b) a combination of 1) a peroxidase enzyme, 2) a peroxide compound, 3) an amino substituted acylhydrazide which generates chemiluminescence upon reaction with the peroxide compound and the peroxidase enzyme and 4) a protected enhancer compound which has the formula ArOX wherein X is a leaving group which is reactive with the hydrolytic enzyme and Ar is a non-interfering aromatic ring group which can contain S, O or N in the ring, wherein the hydrolytic enzyme reacts with the protected enhancer compound to remove X and form an enhancer compound ArOH and thereby enhances the level of chemiluminescence without the enhancer compound characterised in that the combination also comprises 5) a suppressing agent which retards generation of chemiluminescence before mixing of the hydrolytic enzyme.

The present invention further relates to this method, additionally comprising measuring the chemiluminescence produced upon mixing and relating the chemiluminescence to the quantity of the hydrolytic enzyme. The present invention particularly involves a method of generating a catalyst or cycling agent by the action of a first enzyme which enhances or amplifies the action of a second enzyme which, in turn, catalyzes a chemiluminescent reaction. The invention also relates to the use of this method to detect for first enzyme with high sensitivity. Further, the invention relates to the use of the method to detect and quantitate various biological molecules which are bound to this enzyme by chemical bonds or through physical interactions. The intensity of the resulting chemiluminescence provides a direct measure of the quantity of labeled organic or biological molecule. For example, the method may be used to detect haptens, antigens and antibodies by the technique of immunoassay proteins by Western blotting, DNA and RNA by Southern and Northern blotting, respectively. The method may also be used to detect DNA in DNA sequencing applications.

Preferably the compound is a phenolic compound of the formula 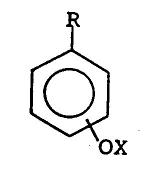 wherein R is a non-interfering group which may be heterocyclic and may also be joined to other parts of the benzene ring.

Thus R can be selected from the group consisting of a halogen (Cl, Br, Fl or I), alkyl containing 1 to 30 carbon atoms (which can be cyclic), aryalkyl containing 1 to 30 carbon atoms and wherein alkylaryl or aralkyl can be substituted with halogen, O, N or S in place of a carbon.

Further the present invention relates to a kit for use in the detection of hydrolytic enzyme in a assay which comprises 1) a peroxide compound, 2) a peroxidase enzyme, 3) an amino substituted acylhydrazide which generates light upon reaction with the peroxide compound and the peroxidase enzyme, and 4) a protected enhancer compound which has the formula ArOX wherein X is a leaving group which is reactive with the hydrolytic enzyme and Ar is a non-interfering aromatic ring group which may contain N, O or S in the ring wherein the hydrolytic enzyme reacts with the protected enhancer compound to remove X and form an enhancer compound and thereby enhance the level of light produced by the amino substituted acylhydrazide as compared to the level of light without the enhancer compound characterised by comprising also 5) a suppressing agent characterised in that the combination comprises which retards generation of chemiluminescence before mixing of the hydrolytic enzyme.

Finally the present invention relates to a solution comprising 1) a peroxide compound, 2) a peroxidase enzyme, 3) an amino substituted acylhydrazide which generates light upon reaction with the peroxide compound and the peroxidase enzyme, and 4) a protected enhancer compound which has the formula ArOX wherein X is a leaving group which is reactive with the hydrolytic enzyme and Ar is a non-interfering aromatic ring group which can contain N, O or S in the ring wherein the hydrolytic enzyme reacts with the protected enhancer compound to remove X and form an enhancer compound and thereby enhance the level of light produced by the amino substituted acylhydrazide as compared characterised by comprising also 5) a suppressing agent characterised in that the combination comprises which retards generation of chemiluminescence before mixing of hydrolytic enzyme with the composition.

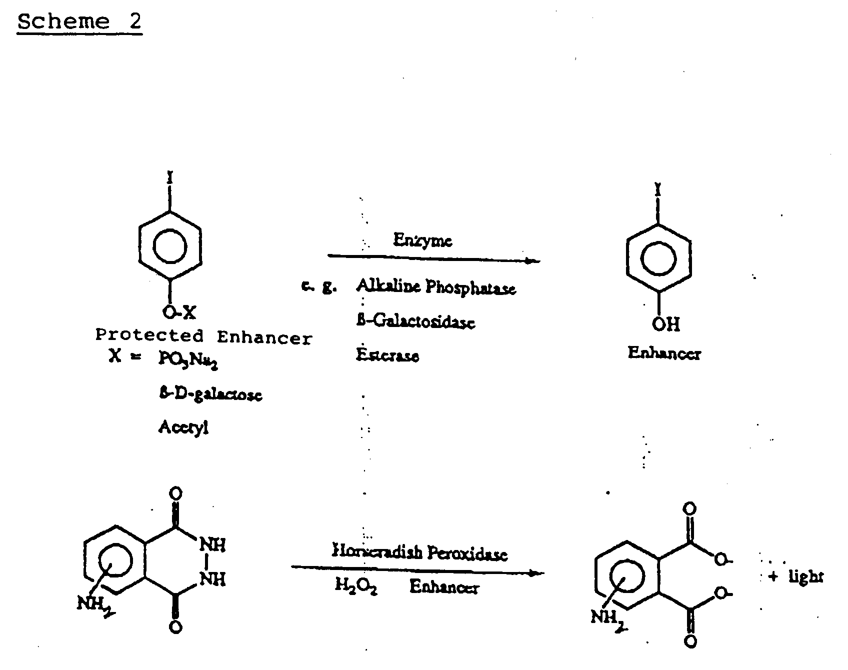

Removal of an X group from a protected enhancer chemical compound by a hydrolytic enzyme generates an enhancer compound, such as a phenolic compound, which greatly enhances the ability of a second enzyme, namely horseradish peroxidase to catalyze the oxidation of luminol with hydrogen peroxide to produce light. Several examples of masked or protected enhancer compounds (ArOX) are shown with specific leaving groups X. Various specific enzymes react with the protected enhancer compounds by cleaving the X group and liberating the arylhydroxy compound or aryloxide ion. Possible X groups include any chemical leaving group which is stable under the conditions of use and may be subsequently removed by treatment of the labelled molecule bearing the group X with the appropriate enzyme. The corresponding OX groups include but are not limited to alkyl or aryl carboxyl ester, inorganic oxyacid salt, and oxygen-pyranoside.

An integral component of the invention is that an amino-substituted cyclic acylhydrazide, a peroxide compound, a peroxidase enzyme and a suppressing agent may be combined in one solution and stored for later use without generating a large background chemiluminescent signal. This would not be expected in view of the fact that the combination of an amino-substituted cyclic acylhydrazide, hydrogen peroxide and a peroxidase enzyme normally constitute a highly chemiluminescent reaction system. Further, incorporation of the protected catalyst to this mixture may also be accomplished without generating a large background chemiluminescent signal.

The invention relates to a method for generating chemiluminescence by the action of an enzyme or enzyme conjugate with a mixture containing an amino-substituted cyclic acylhydrazide, a peroxide compound, a peroxidase enzyme, a protected enhancer compound (ArOX) and a suppressing agent in an aqueous buffer. The invention also relates to the use of the method for the detection of biological molecules such as haptens, antigens, antibodies, and nucleic acids in solution. The invention also relates to the use of the method for the detection on solid supports such as nitrocellulose or nylon membranes, of proteins in Western blots, DNA in Southern blots and other DNA hybridization assays and RNA in Northern blots.

The present method relates to a protected enhancer compound represented by the formula Ar-OX selected from the compounds in Table 1 with X in place of H in OH and wherein Ar is a non-interfering group selected from an aryl, aralkyl, or heteroaryl group, and wherein X is a chemically labile substituent which is removed by an enzyme so that arylhydroxy compound or aryloxide ion is produced and wherein OX is selected from the group consisting of alkyl or aryl carboxyl ester, inorganic oxyacid salt, and oxygen-pyranoside.

**Table 1**

| Aromatic Enhancer Compound (ArOH) | |
|---|---|
| Firefly luciferin | o-Phenylphenol |
| Dehydroluciferin | p-Hydroxycinnamic acid |
| 6-Hydroxybenzothiazole | o-Hydroxycinnamic acid |
| 2-Cyano-6-hydroxybenzothiazole | 2-Chloro-4-phenylphenol |
| p-Iodophenol | 2-Naphthol |
| o-Iodophenol | 1-Bromo-2-naphthol |
| p-Bromophenol | 1,6-Dibromo-2-naphthol |
| p-Chlorophenol 2,4-Dichlorophenol | p-Hydroxyphenylacetic acid |
| p-Phenylphenol | o-Hydroxyphenylacetic acid |

The present method preferably relates to a protected enhancer compound represented by the formula 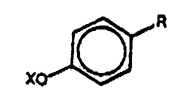 wherein R is selected from the group consisting of I, Ph, or -HC=CH-COOH, wherein X is a chemically labile substituent which is removed by an enzyme so that the phenol or phenolate ion is produced and wherein OX is selected from the group consisting of alkyl or aryl carboxyl ester, inorganic oxyacid salt, and oxygen-pyranoside.

The present method particularly relates to a protected enhancer compound represented by the formula 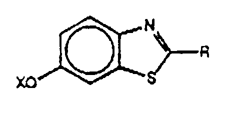 wherein R is selected from the group consisting of H, CN, or 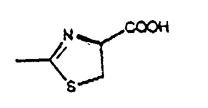 wherein X is a chemically labile substituent which is removed by an enzyme so that the phenol or phenolate ion is produced and wherein OX is selected from the group consisting of alkyl or aryl carboxyl ester, inorganic oxyacid salt, and oxygen-pyranoside.

The present invention involves a solution in an aqueous buffer containing (1) a protected enhancer compound, (2) a peroxide compound wherein the peroxide compound may be hydrogen peroxide, a perborate salt, urea peroxide or an organic peracid, (3) a peroxidase enzyme, wherein the enzyme may be horseradish peroxidase, microperoxidase or lactoperoxidase, (4) an amino-substituted cyclic acylhydrazide, wherein the amino-substituted cyclic acylhydrazide may be selected from the group consisting of 3-aminophthalhydrazide (luminol), 3-(N-alkylamino)phthalhydrazide,3-(N,N-dialkylamino)phthalhydrazide, 6-alkyl-3-aminophthalhydrazide, 4-aminophthalhydrazide(isoluminol), 4-(N-alkylamino)phthalhydrazide, 4-(N,N-dialkylamino)phthalhydrazide, 7-aminophthalhydrazide, 7-(N-alkylamino)naphthalhydrazide 7-(N,N-dialkylamino)naphthalhydrazide and (5) a suppressing agent wherein the agent may be selected from the group consisting of non-fat milk, bovine serum albumin, gelatin or various surfactants including non-ionic, cationic, anionic and zwitterionic surfactants.

### SPECIFIC DESCRIPTION

### Instrumentation

Nuclear magnetic resonance (NMR) spectra were obtained on a General Electric QE300™ spectrometer as solutions in CDCl₃ with tetramethylsilane as internal standard unless noted otherwise. Mass spectra were obtained on either a Kratos MS-80™ or an AEI MS-90™ spectrometer. Weights were obtained on a Mettler AE 163™ analytical balance. Chemiluminescence measurements were performed using either a Turner TD 20e luminometer or a device built in this laboratory which is interfaced to an Apple Macintosh™ computer.

### Materials

Solvents and reagents obtained from various commercial sources were the best available grade and were used without further purification unless noted otherwise. Solvents were dried by distillation from sodium or calcium hydride. Enzymes were purchased from commercial sources and used without further purification. Horseradish peroxidase IgG conjugate was purchased from Cappel Products. The conjugate was used for these studies rather than free commercial enzyme because free enzyme showed phosphatase activity.

### Synthesis of Enzyme Substrates

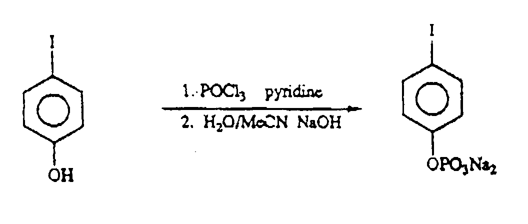

p-Iodophenylphosphate, disodium salt. Pyridine (0.395, g, 5 mmol) was dissolved in 15 mL of CH₂Cl₂. The solution was cooled to about 5°C and stirred while POCl₃ (2.30 g, 15 mmol) was added slowly. Next, a solution of p-iodophenol (1.10 g, 5 mmol) in 10 mL of CH₂Cl₂ was added dropwise over a 5 minute period. The cooling bath was removed and stirring continued for 30 minutes. The solvents were removed under reduced pressure and 15 mL of CH₃CN was added to dissolve the solids. A solution of NaOH (0.80 g, 20 mmol) in 1 mL of water was added dropwise with stirring causing a white precipitate. After standing 10 minutes, the solids were collected and washed with a large volume of CH₃CN followed by acetone and dried in the air.

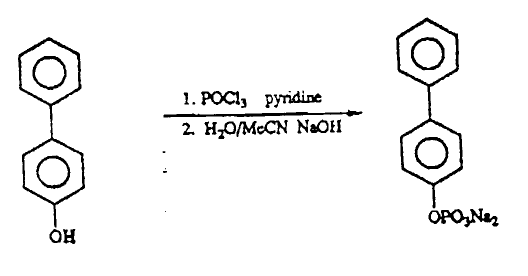

p-Phenylphenol phosphate, disodium salt. Pyridine (0.395 g, 5 mmol) was dissolved in 15 mL of CH₂Cl₂. The solution was cooled to about 5°C and stirred while POCl₃ (2.30 g, 15 mmol) was added slowly. Next, a solution of p-phenylphenol (0.85 g, 5 mmol) in 10 mL of CH₂Cl₂ was added dropwise over a 5 minute period. The cooling bath was removed and stirring continued for 30 minutes. The solvents were removed under reduced pressure and 15 mL of CH₃CN was added to dissolve the solids. A solution of NaOH (0.80 g, 20 mmol) in 1.2 mL of water was added dropwise with stirring causing a white precipitate. After standing 10 minutes, the solids were collected and washed with a large volume of CH₃CN followed by acetone and dried in the air.

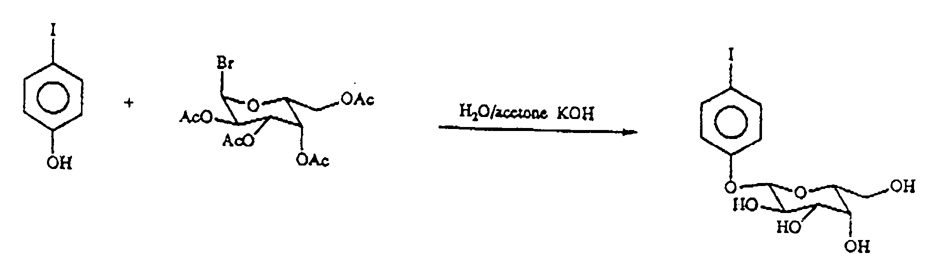

p-Iodophenyl-beta-D-galactopyranoside. p-Iodophenol (1g, 4.5 mmol) dissolved in 3 mL of acetone was treated with 3 mL of 5 M KOH (aq). Acetobromogalactose (5.6 g, 13.6 mmol) was added in portions to the stirred solution. Initially a 4.1 g portion was added with 1 ml of 5 M KOH. At two-three hour intervals 0.5 g portions of acetobromogalactose was added accompanied by 0.5 ml of 5 M KOH until a total of 5.6 g (3 eq.) were added. Stirring was continued overnight. Water was added and the solution extracted with methylene chloride followed by ethyl acetate. The combined organic layers were evaporated and the crude product purified by column chromatography on silica using 30% ethyl acetate in methanol to remove unreacted sugar. Removal of solvents from the appropriate fractions produced the tetraacetate ester of the desired compound. Hydrolysis to the desired compound was achieved by dissolving a 300 mg portion in 2 mL of acetone and stirring with 650 µL of 10 M KOH overnight. The acetone was evaporated and 30 mL of water added. Ammonium chloride was added to neutralize the pH and the resulting solution extracted with ethyl acetate. Evaporation of the ethyl acetate after column chromatography on silica with 1:1 ethyl acetate/methanol.

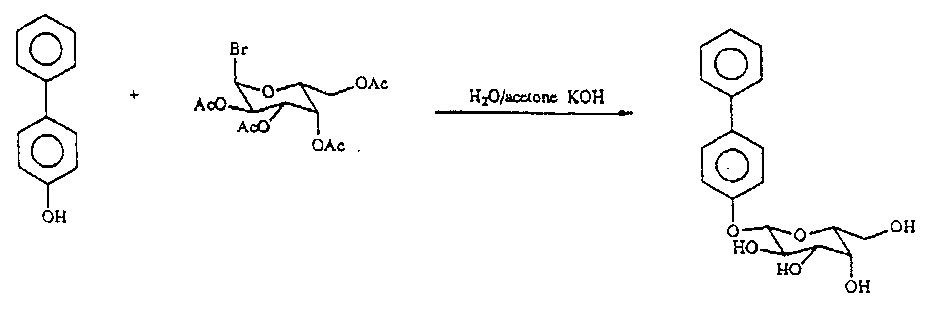

p-Phenylphenol-beta-D-galactopyranoside. p-Phenylphenol (0.25 g, 1.5 mmol) dissolved in 1.5 mL of acetone was treated with 0.5 mL of 10 M KOH (aq). Acetobromogalactose (1.8 g, 4.4 mmol) was added in portions to the stirred solution. Initially a 1.5 g portion was added with 0.5 ml of 10 M KOH. After two hours a 0.3 g portion of acetobromogalactose was added accompanied by 0.5 ml of 10 M KOH. Stirring was continued overnight. TLC indicated that the starting material was consumed. Another 1 mL of 10 M KOH was added and stirring continued for 1 day. The acetone was evaporated and the residue extracted 5 times with ethyl acetate. The combined organic layers were dried, evaporated and the crude product purified by column chromatography on silica using 30% methanol in ethyl acetate.

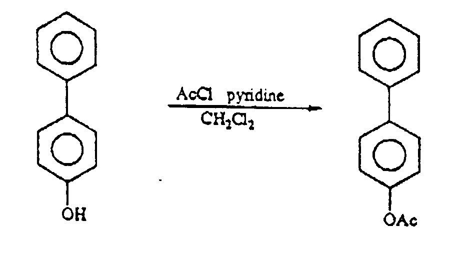

p-Phenylphenol acetate. Pyridine (2 mL) was dissolved in 15 mL of CH₂Cl₂. The solution was cooled to about 5°C and stirred while acetyl chloride (1.8 g, 4.4 mmol) was added slowly. Next, a suspension of p-phenylphenol (0.85 g, 5 mmol) in 20 mL of CH₂Cl₂ was added dropwise over a 15 minute period. The cooling bath was removed and stirring continued for 30 minutes. The solvents were removed under reduced pressure and 50 mL of ethyl acetate was added to dissolve the solids. The solution was extracted with water, dried over Na₂SO₄ and evaporated under reduced pressure.

### Chemiluminescent Detection of Label Enzymes

### Example 1

A typical reaction solution for amplified detection of alkaline phosphatase consists of

| | |
|---|---|
| 2-methyl-2-amino-1-propanol buffer | 0.1M, pH 9.4 |
| Mg(OAc)₂ | 8.8 x 10⁻⁴ M |
| H₂O₂ | 0.3% (v/v) |
| luminol | 1 x 10⁻³ M |
| p-phenylphenol phosphate | 1 x 10⁻³ M |
| horseradish peroxidase-IgG | (1x10⁻¹⁰ - 1x10⁻¹⁸mol) |

Typically, 200 µL of the reagent was transferred into a polypropylene tube placed in a Turner TD 20e or other suitable luminometer. The background luminescence was measured for a period of 5 minutes after which time a 5 µL aliquot of an alkaline phosphatase solution was added. Figure 1 shows a plot of light intensity vs. time produced in this reaction at 25°C. This method allowed the measurement of as little as 6 x 10⁻¹⁸ mol of alkaline phosphatase in 205 µL with a signal/background ratio of 4.

### Example 2

The solution of Example 1 was used with the addition of 0.1% non-fat dry milk (NFM) and the results were similar to Example 1 except that the background was significantly reduced.

### Example 3

The solution of Example 1 was used with the addition of 5% of I-Block solution and the results were similar to Example 1 except that the background was significantly reduced.

### Example 4

The solution of Example 1 was used with the substitution of p-iodophenyl phosphate for p-phenylphenol phosphate and the results were similar to Example 1.

### Example 5

A typical reagent solution for amplified detection of beta-galactosidase consists of

| | |
|---|---|
| phosphate buffer | 0.1M, pH 7.6 |
| Mg(OAc)₂ | 8.8 x 10⁻⁴ M |
| H₂O₂ | 0.3% (v/v) |
| luminol | 1 x 10⁻³ M |
| p-phenylphenol galactoside | 1 x 10⁻³ |
| horseradish peroxidase-IgG | 10⁻¹⁰ - 10⁻¹⁸ mol) |
| non-fat dry milk (NFM) | 0.1% (w/v) |

Typically, 200 µL of the reagent was transferred into a polypropylene tube placed in a Turner TD 20e or other suitable luminometer. The background luminescence was measured for a period of 5 minutes after which time a 5 µL aliquot of a beta-galactosidase solution was added. Emission of chemiluminescence began immediately, reaching a maximum in less than 5 minutes, the intensity being proportional to the amount of beta-galactosidase. The light intensity decayed slowly over more than one hour.

### Example 6

The solution of Example 5 was used with the substitution of p-iodophenyl galactoside for p-phenylphenol galactoside and the results were similar to Example 5.

### Example 7

A typical reagent solution for amplified detection of esterase consists of

| | |
|---|---|
| phosphate buffer, | 0.1 M, pH 7.6 |
| Mg(OAc)₂ | 8.8 x 10⁻⁴ M |
| H₂O₂ | 0.3 % (v/v) |
| luminol | 1 x 10⁻³ M |
| p-phenylphenol acetate | 1 x 10⁻³ M |
| horseradish peroxidase-IgG | 10⁻¹⁰ - 10⁻¹⁸ mol |
| non-fat dry milk (NFM) | 0.1% (w/v) |

Typically, 200 µL of the reagent was transferred into a polypropylene tube placed in a Turner TD 20e or other suitable luminometer. The background luminescence was measured for a period of 5 minutes after which time a 5 µL aliquot of an esterase solution was added. Emission of chemiluminescence began immediately, reaching a maximum in less than 5 minutes, the intensity being proportional to the amount of esterase. The light intensity decayed slowly over more than one hour.

### Chemiluminescent Detection of Proteins by Western Blot

Goat non-immune serum, human whole serum and rabbit anti-goat IgG-alkaline phosphatase conjugate were obtained from Cappel™ Products (Durham, NC). Human transferrin, fractionated goat anti-human transferrin serum, and nitrocellulose membrane were purchased from Sigma Chemical Co. (St. Louis, MO). Each serum and IgG sample was centrifuged at 10,000 g for two minutes and the supernatant was used in the immunological reaction. Immobilon™-P transfer membrane and nylon transfer membrane were obtained from Millipore Corp. (Bedford, MA) and MSI (Micron Separations, Inc., Westboro, MA), respectively. Kodak X-OMAT AR X-ray film (Rochester, NY) was used in the assay procedure.

SDS-PAGE was performed utilizing the buffer system previously described (U. K. Laemmli, Nature, 227, 680 (1970)). The stacking gel was 4.38% acrylamide:0.12% bisacrylamide. The separating gel was 6.81% acrylamide; 0.19% bisacrylamide. Following electrophoresis, the gel was equilibrated for 7-8 minutes with the transfer buffer which contained 20 mM Tris, 153 mM glycine and 20% (v/v) methanol. The gel, sandwiched between a sheet of Immobilon™-P transfer membrane and a sheet of chromatography paper 3MM (Whatman), was placed in the transfer unit (Bio-Rad Laboratories, Richmond, CA). The proteins in the gel were electroeluted for 60-80 minutes at 4°C at a 100 V constant voltage. The membrane was then placed in 50 mM Tris-HCl buffered saline at pH 7.4 (TBS) at 4°C overnight. After this period the membrane was washed with TBS for 15 minutes.

The membrane was treated with 0.05% Tween®-20 in 50mM Tris-HCl buffered saline at pH 7.4 (T-TBS) containing 1% NFM for 1 hour at room temperature. This blocked membrane was incubated for 75 minutes at room temperature with primary antibody (1:500 dilution of goat anti-human transferrin IgG fraction) using T-TBS containing 1% NFM.

The membrane was incubated for 1 hour at room temperature with secondary antibody (1:5000 dilution of rabbit anti-goat IgG-alkaline phosphatase conjugate) in T-TBS containing 1% NFM. After the incubation, the membrane was rinsed and washed four times for 10 minutes each with T-TBS followed by a 10 minute wash in TBS. The membrane was rinsed three times with distilled water.

The washed membrane was soaked in the reagent solution for 5 minutes, drained, placed in a holder (transparency film) and the membrane exposed to X-ray film for a period ranging between 5 seconds and 30 minutes and then developed. Figure 2 shows a chemiluminescent Western slot blot wherein different concentrations of human transferrin bound to an antibody conjugated to alkaline phosphatase on an Immobilon™-P PVdF membrane are contacted with the chemiluminescent reagent solution described in Example 1.

This chemiluminescent method allowed the measurement of 500 femtograms (5 x 10⁻¹³)/slot of human transferrin. Furthermore the method provides a non-fading permanent experimental record on X-ray film and makes it possible to safely and conveniently detect small amounts of protein with very short exposure times.

## Claims

1. A method for generating chemiluminescence which comprises mixing:
(a) a hydrolytic enzyme; and
(b) a combination of 1) a peroxidase enzyme, 2) a peroxide compound, 3) an amino substituted acylhydrazide which generates chemiluminescence upon reaction with the peroxide compound and the peroxidase enzyme and 4) a protected enhancer compound which has the formula ArOX wherein X is a leaving group which is reactive with the hydrolytic enzyme and Ar is a non-interfering aromatic ring group which can contain S, O or N in the ring, wherein the hydrolytic enzyme reacts with the protected enhancer compound to remove X and form an enhancer compound ArOH and thereby enhances the level of chemiluminescence produced by the amino substituted acylhydrazide as compared to the level of chemiluminescence without the enhancer compound characterised in that the combination also comprises 5) a suppressing agent which retards generation of chemiluminescence before mixing of the hydrolytic enzyme.

2. The method of claim 1, further comprising measuring the chemiluminescence produced upon said mixing, and relating the chemiluminescence to the quantity of the hydrolytic enzyme.

3. The method of claim 1 or claim 2 wherein the reaction of the hydrolytic enzyme with the enhancer takes place on a membrane.

4. The method of any preceding claim in which the suppressing agent is selected from proteins and nonionic surfactant.

5. The method of claim 4 wherein the suppressing agent is a protein selected from non fat milk, bovine serum albumin and gelatin.

6. The method of any preceding claim wherein the hydrolytic enzyme is coupled to a biological molecule.

7. The method of claim 6 wherein the hydrolytic enzyme is coupled to an antibody.

8. The method of claim 6 wherein the hydrolytic enzyme is coupled to an antigen.

9. The method of claim 6 wherein the hydrolytic enzyme is coupled to a molecule which is part of a binding pair in as assay.

10. The method of claim 6 wherein the hydrolytic enzyme is coupled to a nucleic acid.

11. The method of any preceding claim wherein OX is selected from the group consisting of alkyl carboxyl ester, carboxyl ester, inorganic oxyacid salt and oxygen pyranoside substituents.

12. The method of any preceding claim wherein Ar is 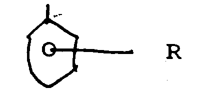 wherein R is selected from the group consisting of a halogen, alkyl containing 1 to 30 carbon atoms, aryl containing 1 to 30 carbon atoms, aralkyl containing 1 to 30 carbon atoms and wherein alkyl, aryl or aralkyl can be substituted with a halogen, R preferably being parasubstituted with respect to OX in ArOX.

13. The method of any preceding claim wherein Ar is p-halophenyl.

14. The method of claim 13 wherein Ar is p-iodophenyl.

15. The method of any of claims 1 to 11 wherein Ar is p-phenylphenyl.

16. The method of any claims 1 to 11 wherein the enhancer compound is selected from the group consisting of firefly luciferin, dehydroluciferin, 6-hydroxybenzothiazole, 2-cyano-6-hydroxybenzothiazole, p-iodophenol, o-iodophenol, p-bromophenol, p-chlorophenol, 2,4-dichlorophenol, p-phenylphenol, o-phenylphenol, p-hydroxycinnamic acid, o-hydroxycinnamic acid, 2-chloro-4-phenylphenol, 2-naphthol, 1-bromo-2-naphthol, 1,6-dibromo-2-naphthol, p-hydroxyphenylacetic acid and o-hydroxyphenylacetic acid.

17. The method of any preceding claim wherein the hydrolytic enzyme is selected from the group consisting of alkaline phosphatase, beta-galactosidase and esterase.

18. The method of any preceding claim wherein the peroxidase enzyme is horseradish peroxidase.

19. The method of any preceding claim wherein the amino-substituted acyl hydrazide is selected from the group consisting of 3-aminophthalhydrazide (luminol), 3-(N-alkylamino)phthalhydrazide, 3-(N,N-dialkylamino)phthalhydrazide, 6-alkyl-3-aminophthalhydrazide, 4-aminophthalhydrazide(isoluminol), 4-(N-alkylamino)phthalhydrazide, 4-(N,N-dialkylamino)phthalhydrazide, 7-aminophthalhydrazide, 7-(N-alkylamino)naphthalhydrazide, 7-(N,N-dialkylamino)naphthalhydrazide.

20. The method of claim 19 wherein the amino substituted acylhydrazide is luminol.

21. A kit for use in the detection of a hydrolytic enzyme in an assay which comprises 1) a peroxide compound, 2) a peroxidase enzyme, 3) an amino substituted acylhydrazide which generates light upon reaction with the peroxide compound and the peroxidase enzyme, and 4) a protected enhancer compound which has the formula ArOX wherein X is a leaving group which is reactive with the hydrolytic enzyme and Ar is a non-interfering aromatic ring group which may contain N, O or S in the ring wherein the hydrolytic enzyme reacts with the protected enhancer compound to remove X and form an enhancer compound and thereby enhance the level of light produced by the amino substituted acylhydrazide as compared to the level of light without the enhancer compound characterised by comprising also 5) a suppressing agent characterised in that the combination comprises which retards generation of chemiluminescence before mixing of the hydrolytic enzyme.

22. A kit according to claim 21 comprising further a hydrolytic enzyme and a membrane.

23. A kit according to claim 21 or 22 in which the suppressing agent is selected from proteins and nonionic surfactants.

24. A kit according to claim 23 in which the suppressing agent is a protein selected from non fat milk, bovine serum albumin and gelatin.

25. A kit according to any of claims 21 to 24 comprising a hydrolytic enzymes coupled to a biological molecule.

26. A kit according to claim 25 in which the biological molecule is an antibody.

27. A kit according to claim 25 in which the biological molecule is an antigen.

28. A kit according to claim 25 in which the biological molecule is part of a binding pair.

29. A kit according to claim 25 in which the biological molecule is a nucleic acid.

30. A kit according to any of claims 21 to 29 wherein OX is selected from the group consisting of alkyl carboxyl ester, carboxyl ester, inorganic oxyacid salt and oxygen pyranoside substituents.

31. A kit according to any of claims 21 to 30 wherein Ar is 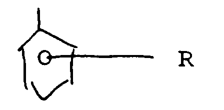 wherein R is selected from the group consisting of a halogen, alkyl containing 1 to 30 carbon atoms, aryl containing 1 to 30 carbon atoms, aralkyl containing 1 to 30 carbon atoms and wherein alkyl, aryl or aralkyl can be substituted with a halogen, R preferably being para substituted with respect to OX in ArOX.

32. A kit according to any of claims 21 to 31 wherein Ar is p-halophenyl.

33. A kit according to claim 32 wherein Ar is p-iodophenyl.

34. A kit according to claim 31 wherein R is phenyl.

35. A kit according to any of claims 21 to 34 wherein the enhancer compound is selected from the group consisting of firefly luciferin, dehydroluciferin, 6-hydroxybenzothiazole, 2-cyan-6-hydroxybenzothiazole, p-iodophenol, o-iodophenol, p-bromophenol, p-chlorophenol, 2,4-dichlorophenol, p-phenylphenol, o-phenylphenol, p-hydroxycinnamic acid, o-hydroxycinnamic acid, 2-chloro-4-phenylphenol, 2-naphthol, 1-bromo-2-naphthol, 1,6-dibromo-2-naphthol, p-hydroxy-phenylacetic acid and o-hydroxyphenylacetic acid.

36. A kit according to any of claims 21 to 35 containing a hydrolytic enzyme selected from the group consisting of alkaline phosphatase, β-galactosidase and esterase.

37. A kit according to any of claims 21 to 36 in which the peroxidase enzyme is horseradish peroxidase.

38. A kit according to any of claims 21 to 37 wherein the amino-substituted acyl hydrazide is selected from the group consisting of 3-aminophthalhydrazide (luminol), 3-(N-alkylamino)phthalhydrazide, 3-(N,N-dialkylamino)phthalhydrazide, 6-alkyl-3-aminophthalhydrazide, 4-aminophthalhydrazide(isoluminol), 4-(N-alkylamino)phthalhydrazide, 4-(N,N-dialkylamino)phthalhydrazide, 7-aminophthalhydrazide, 7-(N-alkylamino)naphthalhydrazide, 7-(N,N-dialkylamino)naphthalhydrazide.

39. A kit according to claim 38 wherein the amino substituted acylhydrazide is luminol.

40. A kit according to any of claims 21 to 39 in which the peroxide, the amino substituted acyl hydrazide, the protected enhancer, the peroxidase enzyme and the suppressor are present in the same solution in the kit.

41. A solution comprising 1) a peroxide compound, 2) a peroxidase enzyme, 3) an amino substituted acylhydrazide which generates light upon reaction with the peroxide compound and the peroxidase enzyme, and 4) a protected enhancer compound which has the formula ArOX wherein X is a leaving group which is reactive with the hydrolytic enzyme and Ar is a non-interfering aromatic ring group which can contain N, O or S in the ring wherein the hydrolytic enzyme reacts with the protected enhancer compound to remove X and form an enhancer compound and thereby enhance the level of light produced by the amino substituted acylhydrazide as compared to the level of light without the enhancer compound characterised by comprising also 5) a suppressing agent which retards generation of chemiluminescence of the solution containing a peroxide compound, a peroxidase enzyme and an amino substituted acylhydrazide.

42. A solution according to claim 41 in which the suppressing agent is selected from proteins and nonionic surfactants.

43. A solution according to claim 42 in which the suppressing agent is a protein selected from non fat milk, bovine serum albumin and gelatin.

44. A solution according to any of claims 41 to 43 wherein OX is selected from the group consisting of alkyl carboxyl ester, carboxyl ester, inorganic oxyacid salt and oxygen pyranoside substituents.

45. A solution according to any of claims 41 to 44 wherein Ar is 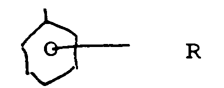 wherein R is selected from the group consisting of a halogen, alkyl containing 1 to 30 carbon atoms, aryl containing 1 to 30 carbon atoms, aralkyl containing 1 to 30 carbon atoms and wherein alkyl, aryl or aralkyl can be substituted with a halogen, R preferably being para substituted with respect to OX in ArOX.

46. A solution according to any of claims 41 to 45 wherein Ar is p-halophenyl.

47. A solution according to claim 46 wherein Ar is p-iodophenyl.

48. A solution according to claim 45 wherein R is phenyl.

49. A solution according to any of claims 41 to 48 wherein the enhancer compound is selected from the group consisting of firefly luciferin, dehydroluciferin, 6-hydroxybenzothiazole, 2-cyano-6-hydroxybenzothiazole, p-iodophenol, o-iodophenol, p-bromophenol, p-chlorophenol, 2,4-dichlorophenol, p-phenylphenol, o-phenylphenol, p-hydroxycinnamic acid, o-hydroxycinnamic acid, 2-chloro-4-phenylphenol, 2-naphthol, 1-bromo-2-naphthol, 1,6-dibromo-2-naphthol, p-hydroxy-phenylacetic acid and o-hydroxyphenylacetic acid.

50. A solution according to any of claims 41 to 49 wherein the amino-substituted acyl hydrazide is selected from the group consisting of 3-aminophthalhydrazide (luminol), 3-(N-alkylamino)phthalhydrazide, 3-(N,N-dialkylamino)phthal-hydrazide, 6-alkyl-3-aminophthalhydrazide, 4-aminophthalhydrazide(isoluminol), 4-(N-alkylamino)phthalhydrazide, 4-(N,N-dialkylamino)phthalhydrazide, 7-aminophthalhydrazide,7-(N-alkylamino)naphthalhydrazide, 7-(N,N-dialkylamino)naphthalhydrazide.

51. A solution according to claim 50 wherein the amino substituted acylhydrazide is luminol.

## Patentansprüche

1. Verfahren zur Erzeugung von Chemilumineszenz, das umfaßt das Mischen von:
(a) einem hydrolytischen Enzym; und
(b) einer Kombination von (1) einem Peroxidase-Enzym, (2) einer Peroxidverbindung, (3) einem Amino-substituierten Acylhydrazid, das bei der Umsetzung mit der Peroxidverbindung und dem Peroxidas-Enzym Chemilumineszenz ausbildet, und (4) einer geschützten Verstärkerverbindung, die die Formel ArOX besitzt, worin X eine austretende Gruppe ist, die mit dem hydrolytischen Enzym reaktiv ist, und Ar eine nicht störende Gruppe eines aromatischen Rings ist, die S, O oder N im Ring enthalten kann, wobei das hydrolytische Enzym mit der geschützten Verstärkerverbindung reagiert, unter Entfernung von X und Ausbildung einer Verstärkerverbindung ArOH, wodurch der Grad der durch das Amino-substituierte Acylhydrazid gebildeten Chemilumineszent im Vergleich zum Grad der Chemilumineszenz ohne Verstärkerverbindung erhöht wird, dadurch gekennzeichnet, daß die Kombination auch (5) ein unterdrückendes Mittel umfaßt, das die Ausbildung der Chemilumineszenz vor dem Mischen mit dem hydrolytischen Enzym hemmt.

2. Verfahren nach Anspruch 1, dadurch gekennzeichnet, daß es außerdem die Bestimmung der beim Mischen ausgebildeten Chemilumineszenz und das in-Beziehung-bringen der Chemilumineszenz mit der Menge des hydrolytischen Enzyms umfaßt.

3. Verfahren nach Anspruch 1 oder Anspruch 2, dadurch gekennzeichnet, daß die Reaktion des hydrolytischen Enzyms mit dem Verstärker an einer Membran stattfindet.

4. Verfahren nach einem der vorhergehenden Ansprüche, dadurch gekennzeichnet, daß das unterdrückende Mittel ausgewählt ist aus Proteinen und nichtionischen oberflächenaktiven Mitteln.

5. Verfahren nach Anspruch 4, dadurch gekennzeichnet, daß das unterdrückende Mittel ein Protein ist, ausgewählt aus fettfreier Milch, Rinderserumalbumin und Gelatine.

6. Verfahren nach einem der vorhergehenden Ansprüche, dadurch gekennzeichnet, daß das hydrolytische Enzym an ein biologisches Molekül gekuppelt ist.

7. Verfahren nach Anspruch 6, dadurch gekennzeichnet, daß das hydrolytische Enzym an einen Antikörper gekuppelt ist.

8. Verfahren nach Anspruch 6, dadurch gekennzeichnet, daß das hydrolytische Enzym an ein Antigen gekuppelt ist.

9. Verfahren nach Anspruch 6, dadurch gekennzeichnet, daß das hydrolytische Enzym an ein Molekül gekuppelt ist, das Teil eines Bindungspaares in einem Assay ist.

10. Verfahren nach Anspruch 6, dadurch gekennzeichnet, daß das hydrolytische Enzym an eine Nucleinsäure gekuppelt ist.

11. Verfahren nach einem der vorhergehenden Ansprüche, dadurch gekennzeichnet, daß OX ausgewählt ist aus der Gruppe bestehend aus Alkylcarbonsäureester, Carbonsäureester, dem Salz einer anorganischen Oxosäure und Sauerstoff-Pyranosid-Substituienten.

12. Verfahren nach einem der vorhergehenden Ansprüche, dadurch gekennzeichnet, daß Ar 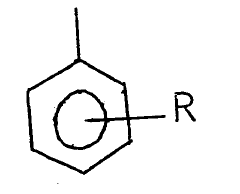 ist, worin R ausgewählt ist aus der Gruppe bestehend aus einem Halogen, einem Alkyl mit 1 bis 30 Kohlenstoffatomen, einem Aryl mit 1 bis 30 Kohlenstoffatomen, einem Aralkyl mit 1 bis 30 Kohlenstoffatomen, und worin Alkyl, Aryl oder Aralkyl durch ein Halogen substituiert sein können, und R im Hinblick auf OX in ArOX vorzugsweise para-substituiert ist.

13. Verfahren nach einem der vorhergehenden Ansprüche, dadurch gekennzeichnet, daß Ar p-Halogenphenyl ist.

14. Verfahren nach Anspruch 13, dadurch gekennzeichnet, daß Ar p-Jodphenyl ist.

15. Verfahren nach einem der Ansprüche 1 bis 11, dadurch gekennzeichnet, daß Ar p-Phenylphenyl ist.

16. Verfahren nach einem der Ansprüche 1 bis 11, dadurch gekennzeichnet, daß die Verstärkerverbindung ausgewählt ist aus der Gruppe bestehend aus Leuchtkäfer-Luciferin, Dehydroluciferin, 6-Hydroxybenzothiazol, 2-Cyano-6-hydroxybenzothiazol, p-Jodphenol, o-Jodphenol, p-Bromphenol, p-Chlorphenol, 2,4-Dichlorphenol, p-Phenylphenol, o-Phenylphenol, p-Hydroxyzimtsäure, o-Hydroxyzimtsäure, 2-Chlor-4-phenylphenol, 2-Naphthol, 1-Brom-2-naphthol, 1,6-Dibrom-2-naphthol, p-Hydroxyphenylessigsäure und o-Hydroxyphenylessigsäure.

17. Verfahren nach einem der vorhergehenden Ansprüche, dadurch gekennzeichnet, daß das hydrolytische Enzym ausgewählt ist aus der Gruppe bestehend aus alkalischer Phosphatase, β-Galactosidase und Esterase.

18. Verfahren nach einem der vorhergehenden Ansprüche, dadurch gekennzeichnet, daß das Peroxidase-Enzym Meerrettichperoxidase ist.

19. Verfahren nach einem der vorhergehenden Ansprüche, dadurch gekennzeichnet, daß das Amino-substituierte Acylhydrazid ausgewählt ist aus der Gruppe bestehend aus 3-Aminophthalsäurehydrazid (Luminol), 3-(N-Alkylamino)phthalsäurehydrazid, 3-(N,N-Dialkylamino)phthalsäurehydrazid, 6-Alkyl-3-aminophthalsäurehydrazid, 4-Aminophthalsäurehydrazid (Isoluminol), 4-(N-Alkylamino)phthalsäurehydrazid, 4-(N,N-Dialkylamino)phthalsäurehydrazid, 7-Aminonaphthalsäurehydrazid, 7-(N-Alkylamino)naphthalsäurehydrazid, 7-(N,N-Dialkylamino)naphthalsäurehydrazid.

20. Verfahren nach Anspruch 19, dadurch gekennzeichnet, daß das Amino-substituierte Acylhydrazid Luminol ist.

21. Reagentiensatz (Kit) zur Verwendung zur Bestimmung eines hydrolytischen Enzyms in einem Assay, der umfaßt (1) eine Peroxidverbindung, (2) ein Peroxidase-Enzym, (3) ein Amino-substituiertes Acylhydrazid, das bei Reaktion mit der Peroxidverbindung und dem Peroxidase-Enzym Licht erzeugt, und (4) eine geschützte Verstärkerverbindung mit der Formel ArOX, worin X eine austretende Gruppe ist, die mit dem hydrolytischen Enzym reaktiv ist, und Ar eine nicht störende Gruppe eines aromatischen Rings, die N, O oder S im Ring enthalten kann, ist, wobei das hydrolytische Enzym mit der geschützten Verstärkerverbindung unter Entfernung von X und Ausbildung einer Verstärkerverbindung reagiert, wodurch der Grad des durch das Amino-substituierte Acylhydrazid gebildeten Lichts im Vergleich zum Grad des Lichts ohne Verstärkerverbindung erhöht wird, dadurch gekennzeichnet, daß er auch (5) ein unterdrückendes Mittel umfaßt, das die Ausbildung von Chemilumineszenz vor dem Mischen mit dem hydrolytischen Enzym hemmt.

22. Reagentiensatz nach Anspruch 21, dadurch gekennzeichnet, daß er außerdem ein hydrolytisches Enzym und eine Membran umfaßt.

23. Reagentiensatz nach Anspruch 21 oder 22, dadurch gekennzeichnet, daß das unterdrückende Mittel ausgewählt ist aus Proteinen und nichtionischen oberflächenaktiven Mitteln.

24. Reagentiensatz nach Anspruch 23, dadurch gekennzeichnet, daß das unterdrückende Mittel ein Protein ist, ausgewählt aus fettfreier Milch, Rinderserumalbumin und Gelatine.

25. Reagentiensatz nach einem der Ansprüche 21 bis 24, dadurch gekennzeichnet, daß er ein an ein biologisches Molekül gekuppeltes hydrolytisches Enzym umfaßt.

26. Reagentiensatz nach Anspruch 25, dadurch gekennzeichnet, daß das biologische Molekül ein Antikörper ist.

27. Reagentiensatz nach Anspruch 25, dadurch gekennzeichnet, daß das biologische Molekül ein Antigen ist.

28. Reagentiensatz nach Anspruch 25, dadurch gekennzeichnet, daß das biologische Molekül Teil eines Bindungspaares ist.

29. Reagentiensatz nach Anspruch 25, dadurch gekennzeichnet, daß das biologische Molekül eine Nucleinsäure ist.

30. Reagentiensatz nach einem der Ansprüche 21 bis 29, dadurch gekennzeichnet, daß OX ausgewählt ist aus der Gruppe bestehend aus Alkylcarbonsäureester, Carbonsäureester, dem Salz einer anorganischen Oxosäure und Sauerstoff-Pyranosid-Substituienten.

31. Reagentiensatz nach einem der Ansprüche 21 bis 30, dadurch gekennzeichnet, daß Ar 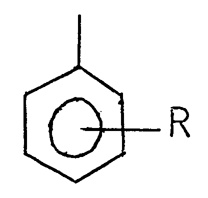 ist, worin R ausgewählt ist aus der Gruppe bestehend aus Halogen, Alkyl mit 1 bis 30 Kohlenstoffatomen, Aryl mit 1 bis 30 Kohlenstoffatomen, Aralkyl mit 1 bis 30 Kohlenstoffatomen, und worin Alkyl, Aryl oder Aralkyl durch ein Halogenatom substituiert sein kann, und R im Hinblick auf OX in ArOX vorzugsweise para-substituiert ist.

32. Reagentiensatz nach einem der Ansprüche 21 bis 31, dadurch gekennzeichnet, daß Ar p-Halogenphenyl ist.

33. Reagentiensatz nach Anspruch 32, dadurch gekennzeichnet, daß Ar p-Jodphenyl ist.

34. Reagentiensatz nach Anspruch 32, dadurch gekennzeichnet, daß R Phenyl ist,

35. Reagentiensatz nach einem der Ansprüche 21 bis 34, dadurch gekennzeichnet, daß die Verstärkerverbindung ausgewählt ist aus der Gruppe bestehend aus Leuchtkäfer-Luciferin, Dehydroluciferin, 6-Hydroxybenzothiazol, 2-Cyano-6-hydroxybenzothiazol, p-Jodphenol, o-Jodphenol, p-Bromphenol, p-Chlorphenol, 2,4-Dichlorphenol, p-Phenylphenol, o-Phenylphenol, p-Hydroxyzimtsäure, o-Hydroxyzimtsäure, 2-Chlor-4-phenylphenol, 2-Naphthol, 1-Brom-2-naphthol, 1,6-Dibrom-2-naphthol, p-Hydroxyphenylessigsäure und o-Hydroxyphenylessigsäure.

36. Reagentiensatz nach einem der Ansprüche 21 bis 35, dadurch gekennzeichnet, daß er ein hydrolytisches Enzym enthält, ausgewählt aus der Gruppe bestehend aus alkalischer Phosphatase, β-Galactosidase und Esterase.

37. Reagentiensatz nach einem der Ansprüche 21 bis 36, dadurch gekennzeichnet, daß das Peroxidase-Enzym Meerrettichperoxidase ist.

38. Reagentiensatz nach einem der Ansprüche 21 bis 37, dadurch gekennzeichnet, daß das Amino-subsituierte Acylhydrazid ausgewählt ist aus der Gruppe bestehend aus 3-Aminophthalsäurehydrazid (Luminol), 3-(N-Alkylamino)phthalsäurehydrazid, 3-(N,N-Dialkylamino)phthalsäurehydrazid, 6-Alkyl-3-aminophthalsäurehydrazid, 4-Aminophthalsäurehydrazid (Isoluminol), 4-(N-Alkylamino)phthalsäurehydrazid, 4-(N,N-Dialkylamino)phthalsäurehydrazid, 7-Aminonaphthalsäurehydrazid, 7-(N-Alkylamino)naphthalsäurehydrazid, 7-(N,N-Dialkylamino)naphthalsäurehydrazid.

39. Reagentiensatz nach Anspruch 38, dadurch gekennzeichnet, daß das Amino-substituierte Acylhydrazid Luminol ist.

40. Reagentiensatz nach einem der Ansprüche 21 bis 39, dadurch gekennzeichnet, daß das Peroxid, das Aminosubstituierte Acylhydrazid, der geschützte Verstärker, das Peroxidase-Enzym und das unterdrückende Mittel im Reagentiensatz in der gleichen Lösung vorhanden sind.

41. Lösung, umfassend: (1) eine Peroxidverbindung, (2) ein Peroxidase-Enzym, (3) ein Amino-substituiertes Acylhydrazid, das bei Reaktion mit der Peroxidverbindung und dem Peroxidase-Enzym Licht erzeugt, und (4) eine geschützte Verstärkerverbindung mit der Formel ArOX, worin X eine austretende Gruppe ist, die mit dem hydrolytischen Enzym reaktiv ist, und Ar eine nicht störende Gruppe eines aromatischen Rings, die N, O oder S im Ring enthalten kann, ist, wobei das hydrolytische Enzym mit der geschützten Verstärkerverbindung unter Entfernung von X und Ausbildung einer Verstärkerverbindung reagiert, wodurch der Grad des durch das Amino-substituierte Acylhydrazid gebildeten Lichts im Vergleich zum Grad des Lichts ohne Verstärkerverbindung erhöht wird, dadurch gekennzeichnet, daß sie außerdem (5) ein unterdrückendes Mittel umfaßt, daß die Ausbildung einer Chemilumineszenz der eine Peroxidverbindung, ein Peroxidase-Enzym und ein Amino-substituiertes Acylhydrazid enthaltenden Lösung hemmt.

42. Lösung nach Anspruch 41, dadurch gekennzeichnet, daß das unterdrückende Mittel ausgewählt ist aus Proteinen und nichtionischen oberflächenaktiven Mitteln.

43. Lösung nach Anspruch 42, dadurch gekennzeichnet, daß das unterdrückende Mittel ein Protein ist ausgewählt aus fettfreier Milch, Rinderserumalbumin und Gelatine.

44. Lösung nach einem der Ansprüche 41 bis 43, dadurch gekennzeichnet, daß OX ausgewählt ist aus der Gruppe bestehend aus Alkylcarbonsäureester, Carbonsäureester, einem Salz einer anorganischen Oxosäure und Sauerstoff-Pyranosid-Substituenten.

45. Lösung nach einem der Ansprüche 41 bis 44, dadurch gekennzeichnet, daß Ar 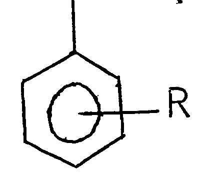 ist, worin R ausgewählt ist aus der Gruppe bestehend aus Halogen, Alkyl mit 1 bis 30 Kohlenstoffatomen, Aryl mit 1 bis 30 Kohlenstoffatomen, Aralkyl mit 1 bis 30 Kohlenstoffatomen, und worin Alkyl, Aryl oder Aralkyl durch ein Halogenatom substituiert sein kann, und R im Hinblick auf OX in ArOX vorzugsweise para-substituiert ist.

46. Lösung nach einem der Ansprüche 41 bis 45, dadurch gekennzeichnet, daß Ar p-Halogenphenyl ist.

47. Lösung nach Anspruch 46, dadurch gekennzeichnet, daß Ar p-Jodphenyl ist.

48. Lösung nach Anspruch 45, dadurch gekennzeichnet, daß R Phenyl ist.

49. Lösung nach einem der Ansprüche 41 bis 48, dadurch gekennzeichnet, daß die Verstärkerverbindung ausgewählt ist aus der Gruppe bestehend aus Leuchtkäfer-Luciferin, Dehydroluciferin, 6-Hydroxybenzothiazol, 2-Cyano-6-hydroxybenzothiazol, p-Jodphenol, o-Jodphenol, p-Bromphenol, p-Chlorphenol, 2,4-Dichlorphenol, p-Phenylphenol, o-Phenylphenol, p-Hydroxyzimtsäure, o-Hydroxyzimtsäure, 2-Chlor-4-phenylphenol, 2-Naphthol, 1-Brom-2-naphthol, 1,6-Dibrom-2-naphthol, p-Hydroxyphenylessigsäure und o-Hydroxyphenylessigsäure.

50. Lösung nach einem der Ansprüche 41 bis 49, dadurch gekennzeichnet, daß das Amino-substituierte Acylhydrazid ausgewählt ist aus der Gruppe bestehend aus 3-Aminophthalsäurehydrazid (Luminol), 3-(N-Alkylamino)phthalsäurehydrazid, 3-(N,N-Dialkylamino)phthalsäurehydrazid, 6-Alkyl-3-aminophthalsäurehydrazid, 4-Aminophthalsäurehydrazid (Isoluminol), 4-(N-Alkylamino)phthalsäurehydrazid, 4-(N,N-Dialkylamino)phthalsäurehydrazid, 7-Aminonaphthalsäurehydrazid, 7-(N-Alkylamino)naphthalsäurehydrazid, 7-(N,N-Dialkylamino)naphthalsäurehydrazid.

51. Lösung nach Anspruch 50, dadurch gekennzeichnet, daß das Amino-substituierte Acylhydrazid Luminol ist.

## Revendications

1. Procédé pour générer une chimioluminescence, qui comprend le mélange de :
(a) une enzyme hydrolytique ; et
(b) une combinaison de 1) une enzyme peroxydase, 2) un composé peroxyde, 3) un acylhydrazide à substitution amino qui génère une chimioluminescence lors d'une réaction avec le composé peroxyde et l'enzyme peroxydase et 4) un composé amplificateur protégé de formule ArOX où X est un groupe partant qui est réactif avec l'enzyme hydrolytique et Ar est un groupe cycloaromatique non interférant qui peut contenir S, O ou N dans le cycle,
dans lequel l'enzyme hydrolytique réagit avec le composé amplificateur protégé pour éliminer X et former un composé amplificateur ArOH et ainsi amplifier le niveau de chimioluminescence produit par l'acylhydrazide à substitution amino par comparaison avec le niveau de chimioluminescence sans le composé amplificateur, caractérisé en ce que la combinaison comprend aussi 5) un agent suppresseur qui retarde la génération de chimioluminescence avant mélange de l'enzyme hydrolytique.

2. Procédé selon la revendication 1, comprenant en outre la mesure de la chimioluminescence produite lors dudit mélange, et la mise en rapport de la chimioluminescence avec la quantité de l'enzyme hydrolytique.

3. Procédé selon la revendication 1 ou la revendication 2, dans lequel la réaction de l'enzyme hydrolytique avec l'amplificateur a lieu sur une membrane.

4. Procédé selon l'une quelconque des revendications précédentes, dans lequel l'agent suppresseur est choisi parmi les protéines et un tensioactif non-ionique.

5. Procédé selon la revendication 4, dans lequel l'agent suppresseur est une protéine choisie parmi la protéine de lait dégraissé, la sérumalbumine bovine et la gélatine.

6. Procédé selon l'une quelconque des revendications précédentes, dans lequel l'enzyme hydrolytique est couplée à une molécule biologique.

7. Procédé selon la revendication 6, dans lequel l'enzyme hydrolytique est couplée à un anticorps.

8. Procédé selon la revendication 6, dans lequel l'enzyme hydrolytique est couplée à un antigène.

9. Procédé selon la revendication 6, dans lequel l'enzyme hydrolytique est couplée à une molécule qui constitue une partie d'une paire de liaison dans un dosage.

10. Procédé selon la revendication 6, dans lequel l'enzyme hydrolytique est couplée à un acide nucléique.

11. Procédé selon l'une quelconque des revendications précédentes, dans lequel OX est choisi dans l'ensemble constitué par un ester alkylcarboxylate, un ester carboxylate, un sel d'oxyacide minéral et les substituants oxypyranosides.

12. Procédé selon l'une quelconque des revendications précédentes, dans lequel Ar est 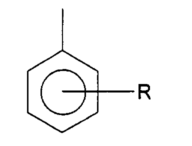 où R est choisi dans l'ensemble constitué par un halogène, un groupe alkyle contenant de 1 à 30 atomes de carbone, un groupe aryle contenant de 1 à 30 atomes de carbone, un groupe aralkyle contenant de 1 à 30 atomes de carbone, et où le groupe alkyle, aryle ou aralkyle peut être substitué par un halogène, R étant de préférence para-substitué par rapport à OX dans ArOX.

13. Procédé selon l'une quelconque des revendications précédentes, dans lequel Ar est un groupe p-halogénophényle.

14. Procédé selon la revendication 13, dans lequel Ar est le groupe p-iodophényle.

15. Procédé selon l'une quelconque des revendications 1 à 11, dans lequel Ar est le groupe p-phénylphényle.

16. Procédé selon l'une quelconque des revendications 1 à 11, dans lequel le composé amplificateur est choisi dans l'ensemble constitué par la luciférine de luciole, la déshydroluciférine, le 6-hydroxybenzothiazole, le 2-cyano-6-hydroxybenzothiazole, le p-iodophénol, le o-iodophénol, le p-bromophénol, le p-chlorophénol, le 2,4-dichlorophénol, le p-phénylphénol, le o-phénylphénol, l'acide p-hydroxycinnamique, l'acide o-hydroxycinnamique, le 2-chloro-4-phénylphénol, le 2-naphtol, le 1-bromo-2-naphtol, le 1,6-dibromo-2-naphtol, l'acide p-hydroxyphénylacétique et l'acide o-hydroxyphénylacétique.

17. Procédé selon l'une quelconque des revendications précédentes, dans lequel l'enzyme hydrolytique est choisie dans l'ensemble constitué par la phosphatase alcaline, la β-galactosidase et l'estérase.

18. Procédé selon l'une quelconque des revendications précédentes, dans lequel l'enzyme peroxydase et la peroxydase de raifort.

19. Procédé selon l'une quelconque des revendications précédentes, dans lequel l'acylhydrazide à substitution amino est choisi dans l'ensemble constitué par le 3-aminophtalhydrazide (luminol), un 3-(N-alkylamino)phtalhydrazide, un 3-(N,N-dialkylamino)phtalhydrazide, un 6-alkyl-3-aminophtalhydrazide, le 4-aminophtalhydrazide (isoluminol), un 4-(N-alkylamino)phtalhydrazide, un 4-(N,N-dialkylamino)phtalhydrazide, le 7-aminophtalhydrazide, un 7-N-alkylamino)naphtalhydrazide, un 7-(N,N-dialkylamino)naphtalhydrazide.

20. Procédé selon la revendication 19, dans lequel l'acylhydrazide à substitution amino est le luminol.

21. Trousse à utiliser dans la détection d'une enzyme hydrolytique dans un dosage qui comprend 1) un composé peroxyde, 2) une enzyme peroxydase, 3) un acylhydrazide à substitution amino qui génère une lumière lors d'une réaction avec le composé peroxyde et l'enzyme peroxydase et 4) un composé amplificateur protégé de formule ArOX où X est un groupe partant qui est réactif avec l'enzyme hydrolytique et Ar est un groupe cycloaromatique non interférant qui peut contenir N, O ou S dans le cycle, dans lequel l'enzyme hydrolytique réagit avec le composé amplificateur protégé pour éliminer X et former un composé amplificateur et ainsi amplifier le niveau de lumière produit par l'acylhydrazide à substitution amino par comparaison avec le niveau de lumière sans le composé amplificateur, caractérisée en ce que qu'elle comprend aussi 5) un agent suppresseur qui retarde la génération de chimioluminescence avant mélange de l'enzyme hydrolytique.

22. Trousse selon la revendication 21, comprenant en outre une enzyme hydrolytique et une membrane.

23. Trousse selon la revendication 21 ou 22, dans laquelle l'agent suppresseur est choisi parmi les protéines et des tensioactifs non-ioniques.

24. Trousse selon la revendication 23, dans laquelle l'agent suppresseur est une protéine choisie parmi la protéine de lait dégraissé, la sérumalbumine bovine et la gélatine.

25. Trousse selon l'une quelconque des revendications 21 à 24, comprenant une enzyme hydrolytique couplée à une molécule biologique.

26. Trousse selon la revendication 25, dans laquelle la molécule biologique est un anticorps.

27. Trousse selon la revendication 25, dans laquelle la molécule biologique est un antigène.

28. Trousse selon la revendication 25, dans laquelle la molécule biologique constitue une partie d'une paire de liaison.

29. Trousse selon la revendication 25, dans laquelle la molécule biologique est un acide nucléique.

30. Trousse selon l'une quelconque des revendications 21 à 29, dans laquelle OX est choisi dans l'ensemble constitué par un ester alkylcarboxylate, un ester carboxylate, un sel d'oxyacide minéral et les substituants oxypyranosides.

31. Trousse selon l'une quelconque des revendications 21 à 30, dans laquelle Ar est 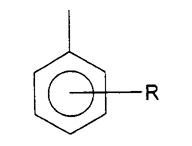 où R est choisi dans l'ensemble constitué par un halogène, un groupe alkyle contenant de 1 à 30 atomes de carbone, un groupe aryle contenant de 1 à 30 atomes de carbone, un groupe aralkyle contenant de 1 à 30 atomes de carbone, et où le groupe alkyle, aryle ou aralkyle peut être substitué par un halogène, R étant de préférence para-substitué par rapport à OX dans ArOX.

32. Trousse selon l'une quelconque des revendications 21 à 31, dans laquelle Ar est un groupe p-halogénophényle.

33. Trousse selon la revendication 32, dans laquelle Ar est le groupe p-iodophényle.

34. Trousse selon la revendication 31, dans laquelle R est le groupe phényle.

35. Trousse selon l'une quelconque des revendications 21 à 34, dans laquelle le composé amplificateur est choisi dans l'ensemble constitué par la luciférine de luciole, la déshydroluciférine, le 6-hydroxybenzothiazole, le 2-cyano-6-hydroxybenzothiazole, le p-iodophénol, le o-iodophénol, le p-bromophénol, le p-chlorophénol, le 2,4-dichlorophénol, le p-phénylphénol, le o-phénylphénol, l'acide p-hydroxycinnamique, l'acide o-hydroxycinnamique, le 2-chloro-4-phénylphénol, le 2-naphtol, le 1-bromo-2-naphtol, le 1,6-dibromo-2-naphtol, l'acide p-hydroxyphénylacétique et l'acide o-hydroxyphénylacétique.

36. Trousse selon l'une quelconque des revendications 21 à 35, contenant une enzyme hydrolytique choisie dans l'ensemble constitué par la phosphatase alcaline, la β-galactosidase et l'estérase.

37. Trousse selon l'une quelconque des revendications 21 à 36, dans laquelle l'enzyme peroxydase et la peroxydase de raifort.

38. Trousse selon l'une quelconque des revendications 21 à 37, dans laquelle l'acylhydrazide à substitution amino est choisi dans l'ensemble constitué par le 3-aminophtalhydrazide (luminol), un 3-(N-alkylamino)phtalhydrazide, un 3-(N,N-dialkylamino)phtalhydrazide, un 6-alkyl-3-aminophtalhydrazide, le 4-aminophtalhydrazide (isoluminol), un 4-(N-alkylamino)phtalhydrazide, un 4-(N,N-dialkylamino)phtalhydrazide, le 7-aminophtalhydrazide, un 7-N-alkylamino)naphtalhydrazide, un 7-(N,N-dialkylamino)naphtalhydrazide.

39. Trousse selon la revendication 38, dans laquelle l'acylhydrazide à substitution amino est le luminol.

40. Trousse selon l'une quelconque des revendications 21 à 39, dans laquelle le peroxyde, l'acylhydrazide à substitution amino, l'amplificateur protégé, l'enzyme peroxydase et le suppresseur sont présents dans la même solution dans la trousse.

41. Solution comprenant 1) un composé peroxyde, 2) une enzyme peroxydase, 3) un acylhydrazide à substitution amino qui génère une lumière lors d'une réaction avec le composé peroxyde et l'enzyme peroxydase et 4) un composé amplificateur protégé de formule ArOX où X est un groupe partant qui est réactif avec l'enzyme hydrolytique et Ar est un groupe cycloaromatique non interférant qui peut contenir N, O ou S dans le cycle, dans laquelle l'enzyme hydrolytique réagit avec le composé amplificateur protégé pour éliminer X et former un composé amplificateur et ainsi amplifier le niveau de lumière produit par l'acylhydrazide à substitution amino par comparaison avec le niveau de lumière sans le composé amplificateur, caractérisée en ce que qu'elle comprend aussi 5) un agent suppresseur qui retarde la génération de chimioluminescence de la solution contenant un composé peroxyde, une enzyme peroxydase et un acylhydrazide à substitution amino.

42. Solution selon la revendication 41, dans laquelle l'agent suppresseur est choisi parmi les protéines et un tensioactif non-ionique.

43. Solution selon la revendication 42, dans laquelle l'agent suppresseur est une protéine choisie parmi la protéine de lait dégraissé, la sérumalbumine bovine et la gélatine.

44. Solution selon l'une quelconque des revendications 41 à 43, dans laquelle OX est choisi dans l'ensemble constitué par un ester alkylcarboxylate, un ester carboxylate, un sel d'oxyacide minéral et les substituants oxypyranosides.

45. Solution selon l'une quelconque des revendications 41 à 44, dans laquelle Ar est 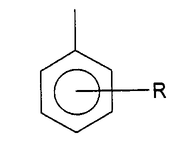 où R est choisi dans l'ensemble constitué par un halogène, un groupe alkyle contenant de 1 à 30 atomes de carbone, un groupe aryle contenant de 1 à 30 atomes de carbone, un groupe aralkyle contenant de 1 à 30 atomes de carbone, et où le groupe alkyle, aryle ou aralkyle peut être substitué par un halogène, R étant de préférence para-substitué par rapport à OX dans ArOX.

46. Solution selon l'une quelconque des revendications 41 à 45, dans laquelle Ar est un groupe p-halogénophényle.

47. Solution selon la revendication 46, dans laquelle Ar est le groupe p-iodophényle.

48. Solution selon la revendication 45, dans laquelle R est le groupe phényle.

49. Solution selon l'une quelconque des revendications 41 à 48, dans laquelle le composé amplificateur est choisi dans l'ensemble constitué par la luciférine de luciole, la déshydroluciférine, le 6-hydroxybenzothiazole, le 2-cyano-6-hydroxybenzothiazole, le p-iodophénol, le o-iodophénol, le p-bromophénol, le p-chlorophénol, le 2,4-dichlorophénol, le p-phénylphénol, le o-phénylphénol, l'acide p-hydroxycinnamique, l'acide o-hydroxycinnamique, le 2-chloro-4-phénylphénol, le 2-naphtol, le 1-bromo-2-naphtol, le 1,6-dibromo-2-naphtol, l'acide p-hydroxyphénylacétique et l'acide o-hydroxyphénylacétique.

50. Solution selon l'une quelconque des revendications 41 à 49, dans laquelle l'acylhydrazide à substitution amino est choisi dans l'ensemble constitué par le 3-aminophtalhydrazide (luminol), un 3-(N-alkylamino)phtalhydrazide, un 3-(N,N-dialkylamino)phtalhydrazide, un 6-alkyl-3-aminophtalhydrazide, le 4-aminophtalhydrazide (isoluminol), un 4-(N-alkylamino)phtalhydrazide, un 4-(N,N-dialkylamino)phtalhydrazide, le 7-aminophtalhydrazide, un 7-N-alkylamino)naphtalhydrazide, un 7-(N,N-dialkylamino)naphtalhydrazide.

51. Solution selon la revendication 50, dans laquelle l'acylhydrazide à substitution amino est le luminol.
